# EUROPÄISCHE PATENTSCHRIFT

(11) **EP 2 776 386 B1**
(45) Veröffentlichungstag und Bekanntmachung des Hinweises auf die Patenterteilung: **20.11.2019**
(21) Anmeldenummer: 12775625.2
(22) Anmeldetag: 19.10.2012
(51) Int. Cl.: C07C 209/10, C07C 211/61

(54) **VERFAHREN ZUR HERSTELLUNG VON ARYLAMINEN**
PROCESS FOR PRODUCING ARYLAMINES
PROCÉDÉ DE PRÉPARATION D'ARYLAMINES

(30) Priorität: 11.11.2011 EP 11008986
(43) Veröffentlichungstag der Anmeldung: 17.09.2014
(73) Patentinhaber: Merck Patent GmbH, 64293 Darmstadt (DE)
(72) Erfinder: KAUFHOLD, Oliver, 64297 Darmstadt (DE); SPREITZER, Hubert, 68519 Viernheim (DE); RIEDMUELLER, Stefan, 60431 Frankfurt am Main (DE)
(86) Internationale Anmeldenummer: PCT/EP2012/004400
(87) Internationale Veröffentlichungsnummer: WO 2013/068075

(56) Entgegenhaltungen:
- WO-A1-2008/133459
- US-A- 5 576 460
- PAIN A J: "MECHANISMS AND MODELS FOR COPPER MEDIATED NUCLEOPHILIC AROMATIC SUBSTITUTION. 2. A SINGLE CATALYTIC SPECIES FROM THREE DIFFERENT OXIDATION STATES OF COPPER IN AN ULLMANN SYNTHESIS OF TRIARYLAMINES", JOURNAL OF THE AMERICAN CHEMICAL SOCIETY, Bd. 109, 1. Januar 1987 (1987-01-01), Seiten 1496-1502, XP000913833, ISSN: 0368-1769, DOI: 10.1021/JA00239A032
- KOJI MATSUO ET AL: "N- ALKYLATION AND N-ARYLATION OF ANILINES STARTING FROM A MILD N-MG REAGENT: ITS ACTIVATION CAUSING THE 'N-C' COUPLING TO EXTEND THE UNIFIED STRUCTURE-REACTIVITY RELATIONSHIP", KOJI MATSUO ET AL, Bd. 7, 1994, Seiten 9-17, XP002689611,
- LOUIE J ET AL: "Discrete High Molecular Weight Triarylamine Dendrimers Prepared by Palladium-Catalyzed Amination", JOURNAL OF THE AMERICAN CHEMICAL SOCIETY, ACS PUBLICATIONS, US, Bd. 119, 1. Januar 1997 (1997-01-01), Seiten 11695-11696, XP002990176, ISSN: 0002-7863, DOI: 10.1021/JA972806D
- DRIVER ET AL: "A Second-Generation Catalyst for Aryl Halide Amination: Mixed Secondary Amines from Aryl Halides and Primary Amines Catalyzed by (DPPF)PdCl2", JOURNAL OF THE AMERICAN CHEMICAL SOCIETY, ACS PUBLICATIONS, US, Bd. 118, Nr. 30, 1. Januar 1996 (1996-01-01), Seiten 7217-7218, XP002296215, ISSN: 0002-7863, DOI: 10.1021/JA960937T
- M. R. BISCOE ET AL: "Electronic effects on the selectivity of Pd-catalyzed C-N bond-forming reactions using biarylphosphine ligands: the competitive roles of amine binding and acidity", ANGEWANDTE CHEMIE. INTERNATIONAL EDITION., Bd. 46, 2007, Seiten 7232-7235, XP002689612, DEVCH VERLAG, WEINHEIM. ISSN: 0570-0833
- SONIA RODRIGUEZ ET AL: "Oxaphosphole-Based Monophosphorus Ligands for Palladium-Catalyzed Amination Reactions", ADVANCED SYNTHESIS & CATALYSIS,, vol. 353, no. 4, 2 March 2011 (2011-03-02) , pages 533-537, XP002643122,
- HUNG-JU YEN ET AL: "Synthesis and unexpected electrochemical behavior of the triphenylamine-based aramids with ortho- and para-trimethyl-protective substitutents", JOURNAL OF POLYMER SCIENCE PART A: POLYMER CHEMISTRY, vol. 48, 2010, pages 5271-5281, John Wiley & Sons, Inc. ISSN: 0887-624X
- Janis Louie ET AL: "SUPPORTING INFORMATION: Discrete High Molecular Weight Triarylamine Dendrimers Prepared by Palladium-Catalyzed Amination", Journal of the American Chemical Society, vol. 119, no. 48, 3 December 1997 (1997-12-03), pages 1-6, XP55382267, US ISSN: 0002-7863, DOI: 10.1021/ja972806d

## Beschreibung

Gegenstand der vorliegenden Erfindung ist ein Verfahren zur Herstellung von Verbindungen enthaltend mindestens eine Arylaminogruppe durch Kupplungsreaktion einer Aminoverbindung mit einer Arylverbindung unter Verwendung einer starken Base.

Das Knüpfen einer Bindung zwischen einem Stickstoffatom und einer Arylgruppe stellt eine Schlüsselreaktion in der organischen Synthese dar. Entsprechend sind Arylamino-Verbindungen oftmals wichtige Zwischenprodukte in mehrstufigen Synthesen. Weiterhin finden Arylamino-Verbindungen Verwendung als pharmazeutische Wirkstoffe sowie als funktionelle Materialien, beispielsweise in elektronischen Vorrichtungen.

In allen Fällen ist das Erzielen einer hohen Ausbeute im Kupplungsschritt sowie das Vermeiden von Nebenprodukten von großer Bedeutung, da anderenfalls Synthesen im industriellen Maßstab nur schwer realisierbar sind. Weiterhin ist es zur Erzielung einer hohen Produktreinheit unabdingbar, dass Nebenreaktionen weitestgehend unterbleiben.

Im Stand der Technik bekannt sind Verfahren zur Synthese von Arylamino-Verbindungen, bei denen die beiden Edukte Aminoverbindung und Arylverbindung unter Übergangsmetallkatalyse, bevorzugt Palladiumkatalyse, in Anwesenheit einer Base zur Reaktion gebracht werden (Hartwig-Buchwald-Kupplung). Solche Verfahren sind unter anderem in US 5576460, in Guram et al., Angew. Chem, Int. Ed. 1995, 43, 1348, in Louie et al., Tet. Lett. 1995, 36, 3609, in Surry et al., Chem. Sci. 2011, 2, 27, in Rodriguez et al., Adv. Synth. Catal. 2011, 353, 533-537, in Louie et al., J. Am. Chem. Soc. 1997, 119, 11695-11696, sowie in Supporting Information, Louie et al., J. Am. Chem Soc. 1997, 119, 11695-11696, offenbart WO2003133459 beschreibt ein ähnliches Verfahren für die gleiche Verbindungen die eine schwächere Base verwenden.

Trotz der insgesamt guten Effizienz dieser Verfahren und ihrer breiten Anwendbarkeit besteht Bedarf an einer Weiterentwicklung der Methode, insbesondere in Hinblick auf langsam reagierende und/oder sterisch anspruchsvolle Edukte. Weiterhin besteht Bedarf an einer Verbesserung des Verfahrens in Hinblick auf Reaktionsdauer, Produktausbeute sowie Verringerung der Bildung von Nebenprodukten, insbesondere der Bildung von defunktionalisierter Arylverbindung und/oder unerwünschtem Kupplungsprodukt mit der eingesetzten Base. Die genannten Probleme treten insbesondere beim Einsatz von sterisch gehinderten Edukten auf, beispielsweise ortho-substituierten Arylverbindungen oder sekundären Aminoverbindungen mit sterisch anspruchsvollen Substituenten.

Unter dem Begriff "sterisch anspruchsvoll" wird im Rahmen der vorliegenden Erfindung verstanden, dass eine Gruppe oder ein Substituent eine große räumliche Ausdehnung aufweist. Das Vorhandensein einer sterisch anspruchsvollen Gruppe führt typischerweise zu einer Verlangsamung von Reaktionen in Positionen, welche benachbart oder in räumlicher Nähe zu der Gruppe liegen. Im Extremfall ist die Reaktion so verlangsamt, dass sie präparativ nicht mehr genutzt werden kann.
Der Begriff ist dem Fachmann auf dem Gebiet der organischen Chemie geläufig. Sterische Hinderung kann durch beliebige Gruppen verursacht werden und ist umso größer, je größer die Raumausdehnung (sterischer Anspruch) der Gruppe ist. Der sterische Anspruch steigt beispielsweise in der Reihe H, Methyl, Ethyl, Isopropyl, tert-Butyl an, so dass H die am wenigsten sterisch anspruchsvolle Gruppe und tert-Butyl die sterisch anspruchvollste Gruppe in dieser Reihe darstellt.

Im Stand der Technik bekannte Versuche zur Weiterentwicklung und Optimierung der Methode betreffen überwiegend die Verwendung neuartiger Ligand-Katalysatorsysteme (vgl. u. a. Surry et al., Chem. Sci. 2011, 2, 27). Wenig Augenmerk wurde hingegen auf einen Wechsel der typischerweise verwendeten Basen gelegt. Gemäß dem Stand der Technik wird in der Hartwig-Buchwald-Reaktion typischerweise NaOtBu eingesetzt (o. g. Literaturstelle oder Kuwano et al., Synlett 2010, 1819). Weiterhin ist die Verwendung von anorganischen Basen wie KOH, NaOH, CS₂CO₃ oder K₃PO₄ im Stand der Technik bekannt (siehe o. g. Literaturstellen).

Überraschenderweise wurde im Rahmen der vorliegenden Erfindung gefunden, dass sich Basen mit einem sehr hohen pKₐ-Wert hervorragend zur Verwendung in übergangsmetallkatalysierten Kupplungsreaktionen wie beispielsweise der Hartwig-Buchwald-Kupplung eignen.

Gegenstand der Erfindung ist somit ein Verfahren zur Herstellung einer Verbindung enthaltend mindestens eine Arylaminogruppe, dadurch gekennzeichnet, dass das Verfahren mindestens eine übergangsmetallkatalysierte Kupplungsreaktion zwischen einer Aminoverbindung und einer Arylverbindung umfasst, bei der eine Base mit einem pKₐ-Wert bezogen auf Dimethylsulfoxid von mindestens 33 eingesetzt wird, wobei die Base aus Amiden mit Alkali- oder Erdalkalimetallgegenion und Organometallverbindungen mit formal negativ geladenem Kohlenstoffatom mit Alkalimetall- oder Erdalkalimetall-Gegenion gewählt ist, und wobei das Verfahren folgendem Schema entspricht: 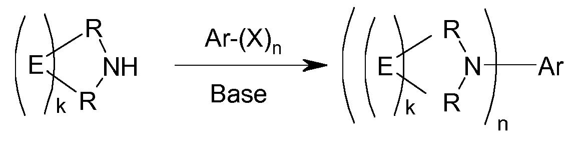 wobei für die auftretenden Symbole und Indices gilt:
- Ar: ist ein aromatisches Ringsystem mit 10 bis 30 aromatischen Ringatomen, das mit einem oder mehreren Resten R¹ substituiert sein kann, und welches mindestens zwei miteinander kondensierte aromatische Ringe aufweist, wobei:
- in der Arylaminogruppe, der eine aromatische Ring in einer ortho-Position zur Bindung an das Stickstoffatom an den anderen aromatischen Ring ankondensiert ist; und
- in der Verbindung Ar-(X)ₙ, der eine aromatische Ring in einer ortho-Position zur Bindung an die X Gruppe an den anderen aromatischen Ring ankondensiert ist;
- E: ist bei jedem Auftreten gleich oder verschieden gewählt aus einer Einfachbindung, C(R¹)₂, C=O, Si(R¹)₂, NR¹, O und S;
- R: ist gleich Phenyl, das durch einen oder mehrere Reste R¹ substituiert sein kann;
- R¹: ist, gleich oder verschieden, bei jedem Auftreten ausgewählt aus H, D, F, Cl, Br, I, B(OR²)₂, CHO, C(=O)R², CR²=C(R²)2, CN, C(=O)OR², C(=O)N(R²)₂, Si(R²)₃, N(R²)₂, NO₂, P(=O)(R²)₂, OSO₂R², OR², S(=O)R², S(=O)₂R², einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenyl- oder Alkinylgruppe mit 2 bis 20 C-Atomen, wobei die oben genannten Gruppen jeweils mit einem oder mehreren Resten R² substituiert sein können und wobei eine oder mehrere CH₂-Gruppen in den oben genannten Gruppen durch -R²C=CR²-, -C≡C-, Si(R²)₂, C=O, C=S, C=NR², -C(=O)O-, -C(=O)NR²-, NR², P(=O)(R²), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches Ringsystem mit 5 bis 30 aromatischen Ringatomen, das jeweils durch einen oder mehrere Reste R² substituiert sein kann, oder eine Aryloxygruppe mit 5 bis 30 aromatischen Ringatomen, die durch einen oder mehrere Reste R² substituiert sein kann, wobei zwei oder mehr Reste R¹ miteinander verknüpft sein können und einen Ring bilden können;
- R²: ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R² miteinander verknüpft sein und einen Ring bilden;
- X: ist bei jedem Auftreten gleich oder verschieden eine beliebige Abgangsgruppe;
- k: ist bei jedem Auftreten gleich oder verschieden 0 oder 1, wobei, wenn k = Null ist, die Gruppe E nicht vorhanden ist;
- i: ist bei jedem Auftreten gleich oder verschieden einen Wert von 0 bis 5, wenn k = Null ist, oder einen Wert von 0 bis 4, wenn k = 1; und
- n: ist 1;
wobei die Verbindung 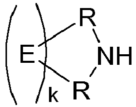 die Aminoverbindung darstellt, die Verbindung der Formel

Ar-(X)ₙ

die Arylverbindung darstellt, und die Verbindung 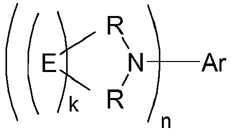 die Verbindung, welche mindestens eine Arylaminogruppe enthält, darstellt,
und wobei ein Katalysator vorhanden ist, wobei der Katalysator eine Verbindung darstellt, welche mindestens ein Metall aus Palladium enthält.

Unter einer Aminoverbindung wird im Rahmen der vorliegenden Erfindung eine wahlweise substituierte organische Verbindung enthaltend mindestens eine Aminogruppe verstanden. Bevorzugt stellt die Aminoverbindung eine Diarylaminoverbindung dar, d. h. eine Verbindung, bei der zwei Arylgruppen an ein Stickstoffatom gebunden sind, welches als dritten Substitutenten ein Wasserstoffatom trägt. Besonders bevorzugte Ausführungsformen von Aminoverbindungen gemäß der vorliegenden Erfindung werden in später folgenden Abschnitten offenbart.

Unter einer Arylverbindung wird im Rahmen der vorliegenden Erfindung eine wahlweise substituierte organische Verbindung enthaltend mindestens ein aromatisches Ringsystem verstanden. Bevorzugte Ausführungsformen von Arylverbindungen gemäß der vorliegenden Erfindung werden in später folgenden Abschnitten offenbart.

Im Rahmen der vorliegenden Anmeldung ist von den Begriffen "Aryl-" und "aromatisch" jeweils "Heteroaryl-" und "heteroaromatisch" mit umfasst, sofern nicht im expliziten Fall eine Unterscheidung getroffen wird.

Ein aromatisches Ringsystem im Sinne dieser Erfindung enthält 5 bis 60 aromatische Ringatome, unter denen sich auch Heteroatome befinden können. Es kann substituiert oder unsubstituiert sein. Die Heteroatome sind bevorzugt ausgewählt aus N, O und/oder S. Unter einem aromatischen Ringsystem im Sinne dieser Erfindung soll ein System verstanden werden, das nicht notwendigerweise nur Arylgruppen enthält, sondern in dem auch mehrere Arylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Arylgruppen über Einfachbindungen miteinander verknüpft sind, als aromatische Ringsysteme im Sinne dieser Erfindung verstanden, wie beispielsweise Systeme wie Biphenyl, Terphenyl oder Diphenyltriazin.

Unter einem aromatischen Ringsystem mit 5 - 60 aromatischen Ringatomen, welches noch jeweils mit Resten wie oben definiert substituiert sein kann und welches über beliebige Positionen am Aromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Eine Arylgruppe im Sinne dieser Erfindung enthält 5 bis 60 aromatische Ringatome; sie kann auch Heteroatome enthalten. Die Heteroatome sind bevorzugt ausgewählt aus N, O und S. Die Arylgruppe kann gemäß der vorliegenden Erfindung substituiert oder unsubstituiert sein. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenden Heteroatome, so gelten diese.

Dabei wird unter einer Arylgruppe entweder ein einfacher aromatischer Cyclus, also Benzol, oder ein einfacher heteroaromatischer Cyclus, beispielsweise Pyridin, Pyrimidin oder Thiophen, oder ein kondensierter (annellierter) aromatischer oder heteroaromatischer Polycyclus, beispielsweise Naphthalin, Phenanthren, Chinolin oder Carbazol verstanden. Ein kondensierter (annellierter) aromatischer oder heteroaromatischer Polycyclus besteht im Sinne der vorliegenden Anmeldung aus zwei oder mehr miteinander kondensierten einfachen aromatischen oder heteroaromatischen Cyclen.

Unter einer Arylgruppe, die jeweils mit den oben genannten Resten substituiert sein kann und die über beliebige Positionen am Aromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol. Unter einer Arylaminogruppe wird im Rahmen der vorliegenden Erfindung eine chemische Gruppe verstanden, in der eine oder mehrere aromatische Ringsysteme, bevorzugt drei aromatische Ringsysteme, an ein Stickstoffatom gebunden sind. Die Arylaminogruppe kann substituiert oder unsubstituiert sein.

Unter einer übergangsmetallkatalysierten Kupplungsreaktion wird im Rahmen der vorliegenden Erfindung eine Reaktion zwischen zwei organischen Verbindungen verstanden, bei der unter Übergangsmetallkatalyse eine Einfachbindung zwischen den beiden Verbindungen gebildet wird. Dies erfolgt unter formaler Abspaltung eines kleinen Moleküls. Bevorzugt treten keine weiteren Reaktionen an den beiden Verbindungen auf. Die Kupplungsreaktion erfolgt erfindungsgemäß zwischen einer Aminoverbindung und einer Arylverbindung, wobei die Arylverbindung bevorzugt eine Abgangsgruppe aufweist. Die Abgangsgruppe der Arylverbindung ist bevorzugt gewählt aus Halogenid, Alkylsulfonat, Arylsulfonat und Diazonium, besonders bevorzugt aus Cl, Br, I, Methylsulfonat, Trifluormethylsulfonat, Phenylsulfonat und Tolylsulfonat. In der Kupplungsreaktion tritt die gebildete Einfachbindung jeweils bevorzugt an die Stelle der N-H-Bindung der Aminoverbindung und an die Stelle der Bindung zur Abgangsgruppe der Arylverbindung. Für eine genauere Erläuterung und Beschreibung bevorzugter Ausführungsformen der Kupplungsreaktion wird auf folgende Abschnitte verwiesen. Erfindungsgemäß bevorzugt ist eine übergangsmetallkatalysierte Kupplungsreaktion gewählt aus der dem Fachmann bekannten Klasse der Hartwig-Buchwald-Reaktionen.

Als pKₐ-Wert bezogen auf Dimethylsulfoxid (DMSO) wird im Rahmen der vorliegenden Anmeldung der in DMSO bestimmte bzw. in diesem System extrapolierte pKₐ-Wert verstanden.

Der pKₐ-Wert ist, wie dem Fachmann allgemein bekannt ist, der negative dekadische Logarithmus des Werts für die Gleichgewichtskonstante der Deprotonierungsreaktion einer Säure. Er stellt somit ein Maß für die Säurestärke dar. Je höher der pKₐ-Wert einer Verbindung ist, umso schwächer sauer ist die betreffende Verbindung. Umgekehrt gilt für die konjugierte Base der Verbindung, dass diese umso stärker basisch ist, je höher der pKₐ-Wert der Verbindung ist.

pK_{A}-Werte können experimentell bestimmt werden über die Gleichgewichtskonstante der Deprotonierungsreaktion. Für eine detaillierte Beschreibung von Verfahren zur experimentellen Bestimmung von pKₐ-Werten wird beispielsweise auf McEwen et al., J. Am. Chem. Soc. 1936, 58, 1124, verwiesen.

Weiterhin sind pK_{A}-Werte einer Vielzahl von Verbindungen dem Fachmann über entsprechende Nachschlagewerke und Tabellen zugänglich. Für Verbindungen, für die keine tabellierten Werte vorliegen, kann vielfach eine Interpolation ausgehend von strukturell ähnlichen Verbindungen erfolgen, so dass auch für solche Verbindungen pK_{A}-Werte erhältlich werden. Alternativ kann der Fachmann den pKₐ-Wert einer nicht tabellierten Verbindung nach dem oben genannten Verfahren experimentell bestimmen.

Gemäß einer bevorzugten Ausführungsform der Erfindung weist die in dem erfindungsgemäßen Verfahren verwendete Base einen pKₐ-Wert auf, welcher mindestens um den Betrag von 6 größer ist als der pKₐ-Wert der Aminoverbindung. Besonders bevorzugt weist sie einen pKₐ-Wert auf, der mindestens um einen Betrag von 8 größer ist als der pKₐ-Wert der Aminoverbindung. Am stärksten bevorzugt ist der pKₐ-Wert mindestens um einen Betrag von 10 größer als der pKₐ-Wert der Aminoverbindung.

Bevorzugt weist die in dem erfindungsgemäßen Verfahren verwendete Base einen pKₐ-Wert bezogen auf Dimethylsulfoxid von mindestens 34 auf, besonders bevorzugt von mindestens 36, ganz besonders bevorzugt von mindestens 38 und am stärksten bevorzugt von mindestens 40.

Bevorzugte Basen zur Verwendung in dem erfindungsgemäßen Verfahren sind Amide mit Alkali- oder Erdalkalimetallgegenion aus Lithiumamid, Lithiumdiisopropylamid (LDA), Lithiumtetramethylpiperidid und Lithiumpyrrolidid ausgewählt. Weiterhin bevorzugt sind Organometallverbindungen mit formal negativ geladenem Kohlenstoffatom mit Alkalimetall- oder Erdalkalimetall-Gegenion aus Alkalimetall-Gegenion, besonders bevorzugt Lithium-Gegenion, ausgewählt. Besonders bevorzugte Basen sind Methyllithium, Ethyllithium, n-Propyllithium, Isopropyllithium, n-Butyllithium, s-Butyllithium, t-Butyllithium, n-Pentyllithium und dessen Isomere, Cyclopentyllithium, n-Hexyllithium und dessen Isomere, Phenyllithium und Benzyllithium.

Die Base wird bevorzugt in einer Menge von 0.5 bis 10 Äquivalenten, bezogen auf die Stoffmenge an eingesetzter Aminoverbindung, eingesetzt. Besonders bevorzugt werden 0.6 bis 5 Äquivalente, ganz besonders bevorzugt 0.7 bis 2 Äquivalente und am stärksten bevorzugt 0.8 bis 1 Äquivalente an Base eingesetzt.

Die Base wird bevorzugt zu Beginn der Reaktion zur Mischung hinzugegeben. Besonders bevorzugt geschieht dies schrittweise, ganz besonders bevorzugt tropfenweise über einen Zeitraum von wenigstens 5 Minuten.

Es soll weiterhin angemerkt werden, dass sich das erfindungsgemäße Verfahren auch zur Synthese von oligomeren oder polymeren Verbindungen, enthaltend mindestens eine Arylaminogruppe, eignet. Das oben gezeigte prinzipielle Reaktionsschema umfassend eine Bindungsknüpfung zwischen einem Stickstoffatom und einer Arylgruppe ist dafür weiterhin gültig.

Bevorzugt wird das erfindungsgemäße Verfahren zur Herstellung von kleinen organischen Verbindungen, d. h. Verbindungen mit einem Molekulargewicht von weniger als 5000 Da, besonders bevorzugt weniger als 3000 Da und ganz besonders bevorzugt weniger als 2000 Da, verwendet.

Bevorzugt ist R¹ bei jedem Auftreten gleich oder verschieden ausgewählt aus H, D, F, CN, Si(R²)₃, N(R²)₂ oder einer geradkettigen Alkyl- oder Alkoxygruppe mit 1 bis 20 C-Atomen oder einer verzweigten oder cyclischen Alkyl- oder Alkoxygruppe mit 3 bis 20 C-Atomen, wobei die oben genannten Gruppen jeweils mit einem oder mehreren Resten R² substituiert sein können und wobei in den oben genannten Gruppen eine oder mehrere CH₂-Gruppen durch -C=C-, -R²C=CR²⁻, Si(R²)₂, C=O, C=NR², -NR²-, -O-, -S-, -C(=O)O- oder -C(=O)NR²- ersetzt sein können, oder ein aromatisches Ringsystem mit 5 bis 20 aromatischen Ringatomen, das jeweils mit einem oder mehreren Resten R² substituiert sein kann, wobei zwei oder mehr Reste R¹ miteinander verknüpft sein können und einen Ring bilden können.

Besonders bevorzugt ist derjenige Rest R¹, der in ortho-Position zur Bindung an das Stickstoffatom gebunden ist, ausgewählt aus Alkylgruppen mit 1 bis 10 C-Atomen, welche mit einem oder mehreren Resten R² substituiert sein können, und Arylgruppen mit 5 bis 18 aromatischen Ringatomen, welche mit einem oder mehreren Resten R² substituiert sein können.

E ist bevorzugt eine Einfachbindung.

X ist bei jedem Auftreten gleich oder verschieden gewählt aus Cl, Br, I, Methylsulfonat, Trifluormethylsulfonat, Phenylsulfonat, Tolylsulfonat und Diazonium.

Eine bevorzugte Ausführungsform für die Aminoverbindung entspricht der folgenden Formel (N) 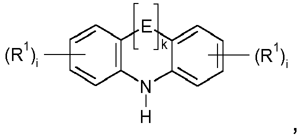 wobei R¹ wie oben definiert ist, E eine Einfachbindung darstellt, k gleich 0 oder 1 ist, und i bei jedem Auftreten gleich oder verschieden einen Wert von 0 bis 5 aufweist und die Zahl der an den Phenylring gebundenen Gruppen R¹ bezeichnet. Bevorzugt weist mindestens ein Index i pro Formel (N) einen Wert von 1 bis 5 auf. Bevorzugt weist die Aminoverbindung der Formel (N) mindestens eine in ortho-Position zur Aminogruppe gebundene Gruppe R¹ auf. Ist k gleich Null, ist die Gruppe E nicht vorhanden.

Besonders bevorzugte Ausführungsformen von Aminoverbindungen sind die im Folgenden gezeigten Verbindungen der Formeln (N-1) bis (N-26) 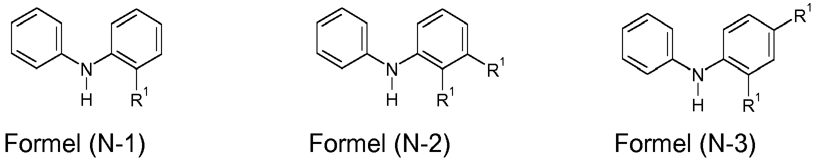 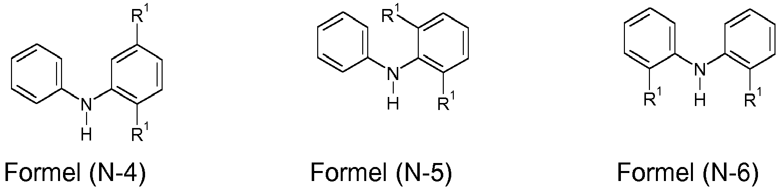 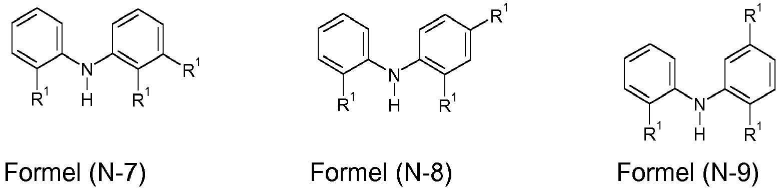 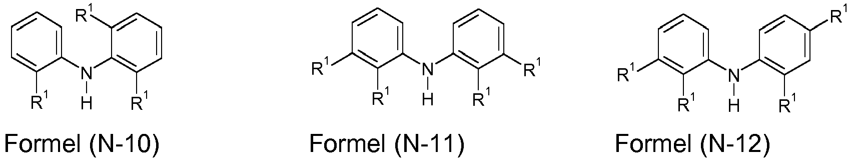 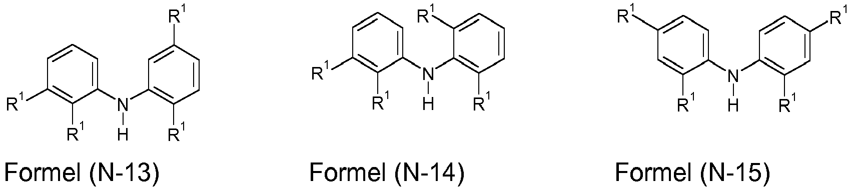 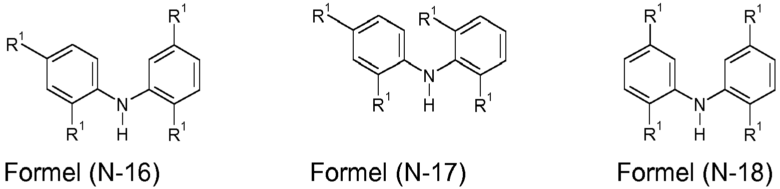 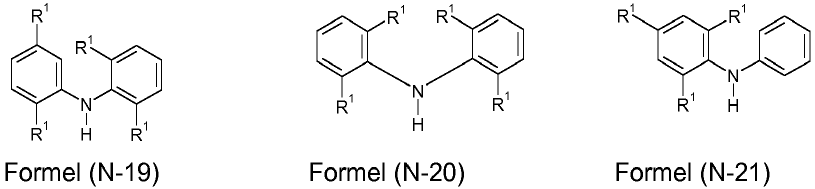 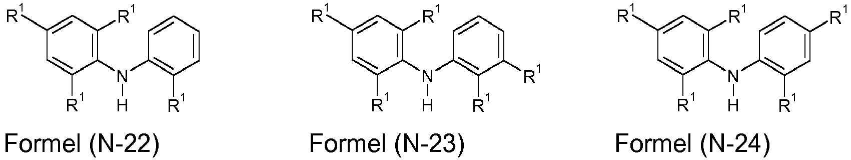 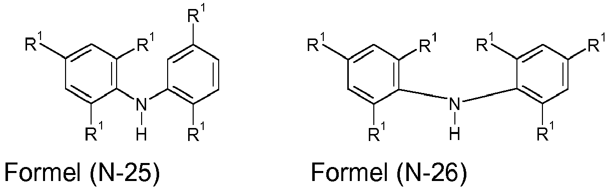 wobei R¹ wie oben definiert ist und ungleich H und D ist.

Weiterhin sind bevorzugte Ausführungsformen von Aminoverbindungen die im Folgenden gezeigten Verbindungen der Formeln (N-27) bis (N-38) 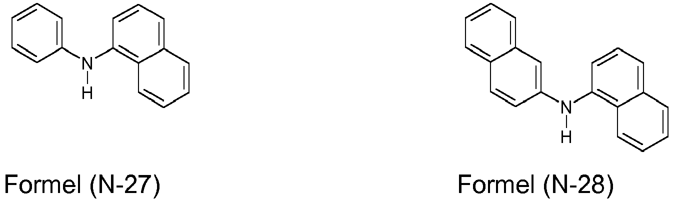 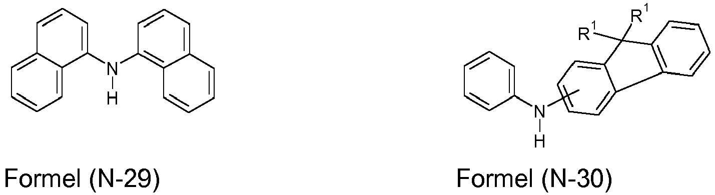 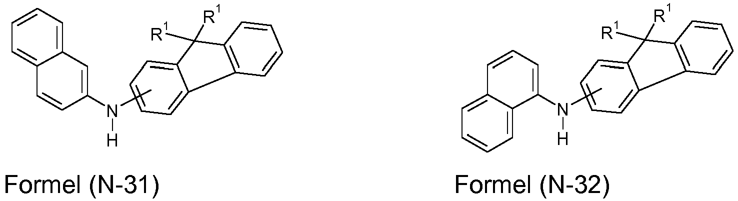 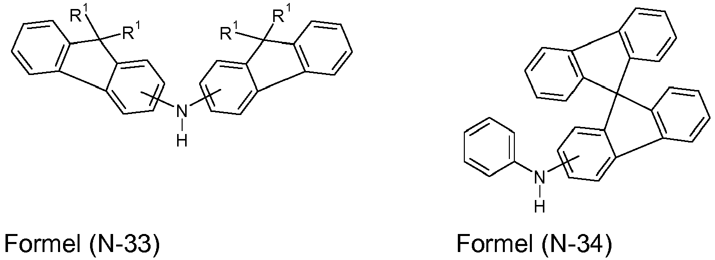 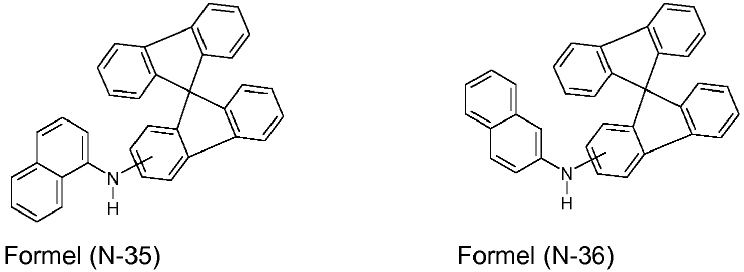 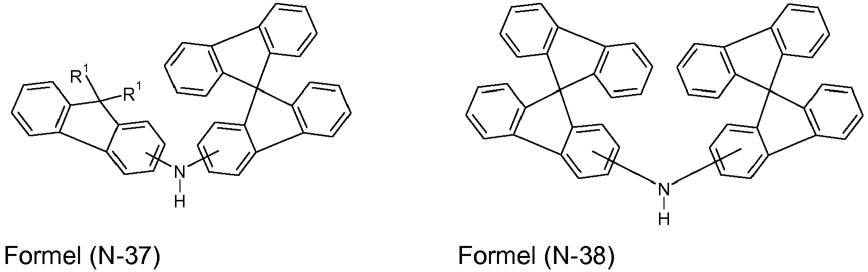 wobei die Verbindungen an allen unsubstituiert dargestellten Positionen mit einem Rest R¹ substituiert sein können, welcher ausgewählt ist aus den oben aufgeführten Ausführungsformen, und wobei die Fluorenyl- und Spirobifluorenylgruppen in beliebiger Position an ihrem aromatischen Sechsring gebunden sein können.

Allgemein sind erfindungsgemäß in den Arylverbindungen Ar-(X)ₙ sterisch anspruchsvolle Gruppen Ar bevorzugt, ebenso wie Gruppen Ar, welche mit sterisch anspruchsvollen Gruppen R¹, bevorzugt in ortho-Position zur Bindung an die Abgangsgruppe X, substituiert sind.

Bevorzugt umfasst Ar als Bestandteil der Arylverbindung Ar-(X)ₙ eine kondensierte Arylgruppe ausgewählt aus Naphthalin, Anthracen, Phenanthren, Pyren, Chrysen, Perylen, Fluoranthen, Benzanthracen, Benzphenanthren, Tetracen, Pentacen, Benzpyren, Acridin und Phenanthridin. Ebenfalls bevorzugt umfasst Ar als Bestandteil der Arylverbindung Ar-(X)ₙ ein aromatisches Ringsystem ausgewählt aus Fluoren, Spirobifluoren, cis- oder trans-Indenofluoren, cis- oder trans-Indolocarbazol, cis- oder trans-Indenocarbazol, cis- oder trans-Monobenzindenofluoren und cis- oder trans-Dibenzindenofluoren.

Besonders bevorzugte Gruppen Ar als Bestandteil der Arylverbindung Ar-(X)ₙ entsprechen den folgenden Formeln (A-1) bis (A-23) 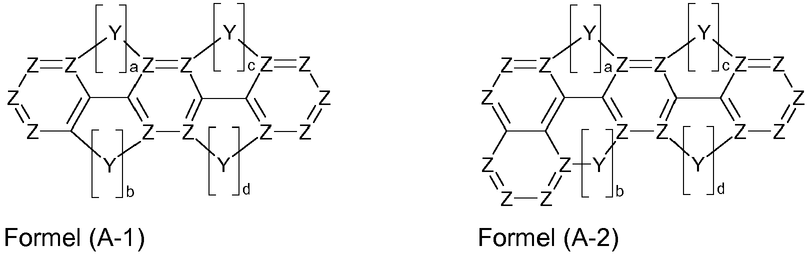 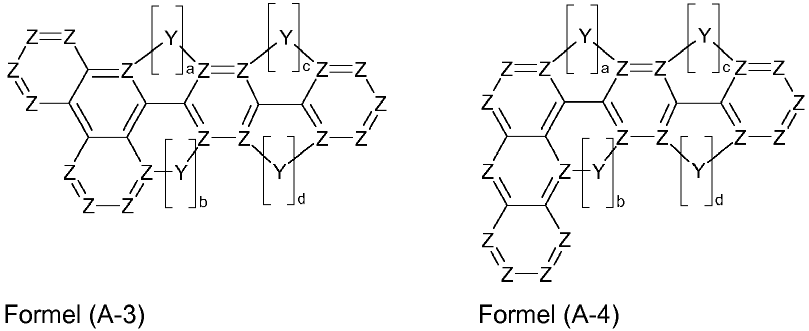 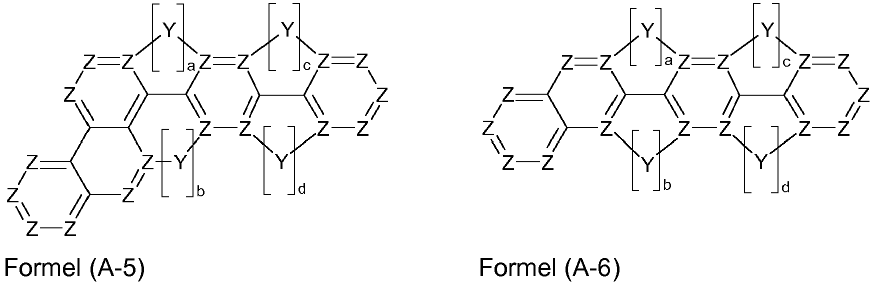 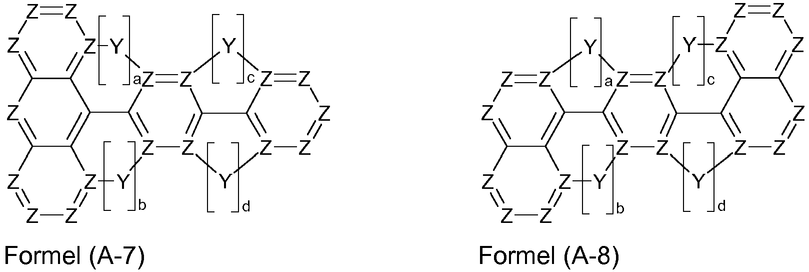 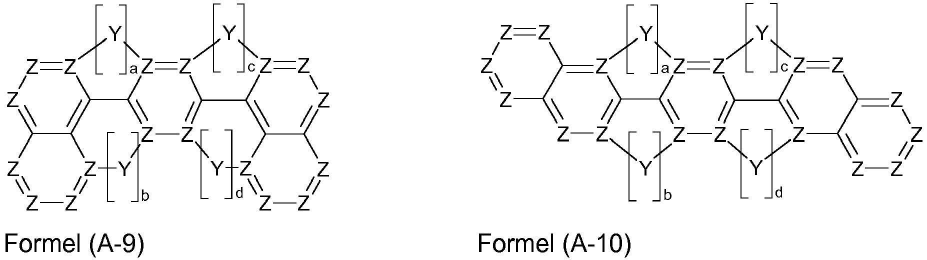 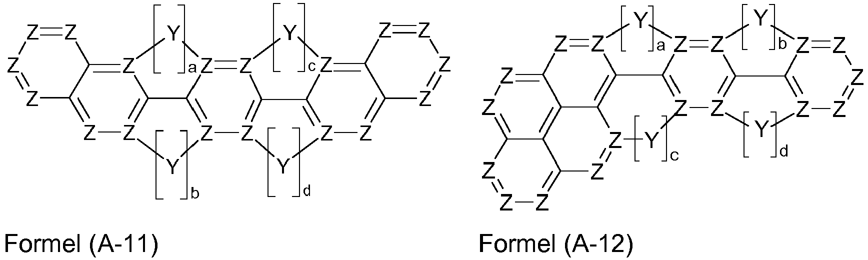 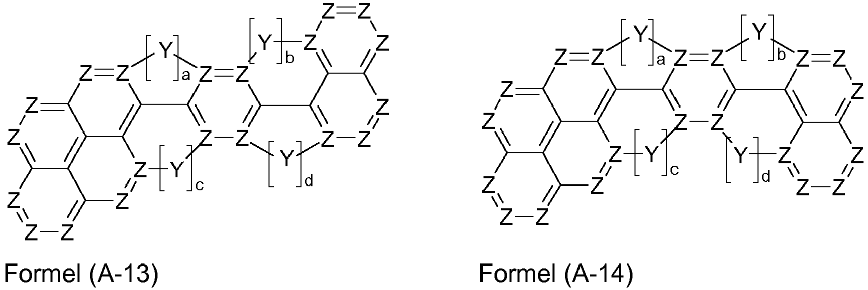 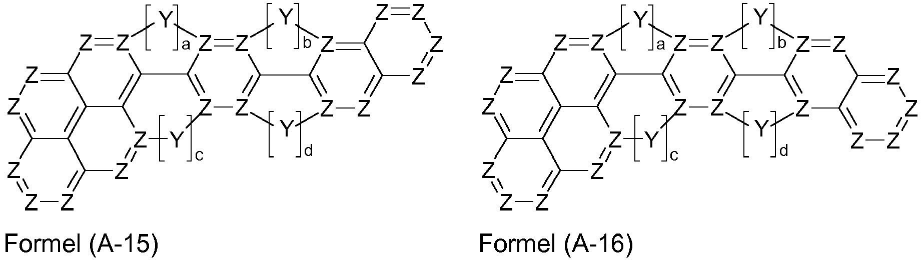 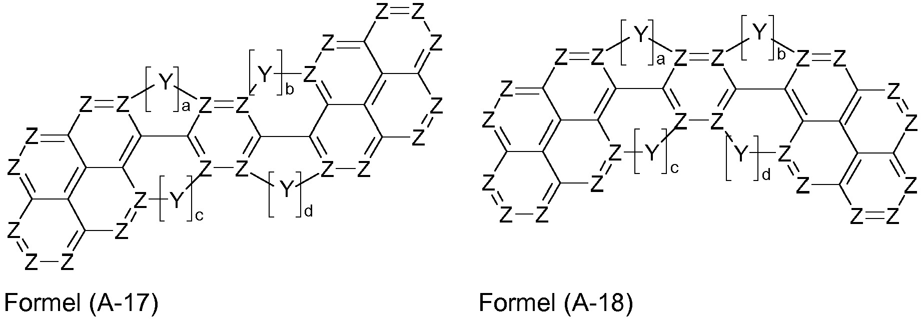 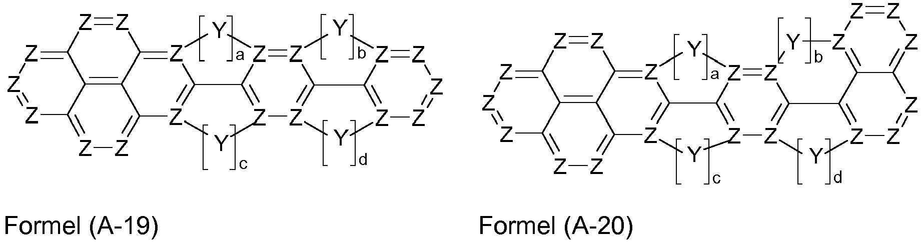 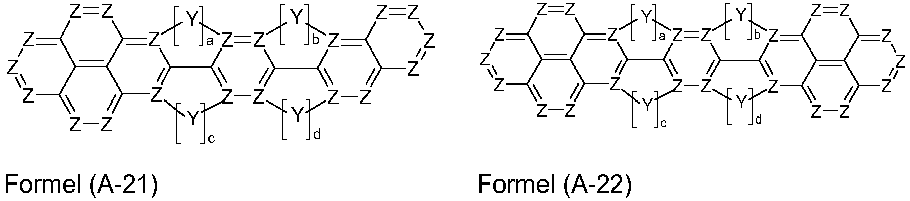 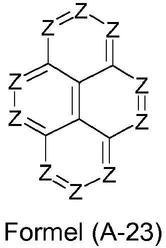 wobei für die auftretenden Symbole und Indices gilt:
Z ist bei jedem Auftreten gleich oder verschieden CR¹ oder N, wenn keine Gruppe X oder Y gebunden ist, und ist gleich C, wenn eine Gruppe X oder Y gebunden ist;
Y ist bei jedem Auftreten gleich oder verschieden ausgewählt aus B(R¹), C(R¹)₂, Si(R¹)₂, C=O, C=NR¹, C=C(R¹)₂, O, S, S=O, SO₂, N(R¹), P(R¹) und P(=O)R¹;
R¹ ist definiert wie oben angegeben; und
a, b, c und d sind bei jedem Auftreten gleich oder verschieden 0 oder 1, wobei die Summe aus a und b größer als Null ist und die Summe aus c und d größer als Null ist.

Die Gruppe X kann in den Arylverbindungen Ar-(X)ₙ an beliebiger Stelle an der Gruppe Ar gebunden sein. In den Gruppen Ar der Formeln (A-1) bis (A-22) ist sie bevorzugt an eine Gruppe Z gebunden.

Die in der Reaktion erhaltenen Verbindungen enthaltend mindestens eine Arylaminogruppe (Produkte) sind dadurch gekennzeichnet, dass an die Stelle der Bindung Ar-(X)ₙ der Arylverbindung eine Bindung Ar-N tritt, wobei N das Stickstoffatom der Aminoverbindung bezeichnet. Bevorzugte Verfahrensprodukte des erfindungsgemäßen Verfahrens sind somit Kombinationen der oben angegebenen bevorzugten Edukte Arylverbindung und Aminoverbindung. Besonders bevorzugt sind Kupplungsprodukte, resultierend aus Kombinationen der Verbindungen gemäß den bevorzugten Formeln (A-1) bis (A-23) und (N-1) bis (N-38).

Im erfindungsgemäßen Verfahren wird ein Übergangsmetall-Katalysator eingesetzt. Dieser kann entweder zu Beginn der Reaktion oder zu einem beliebigen späteren Zeitpunkt zugegeben werden. Bevorzugt ist der Katalysator bereits in der Mischung vorhanden, wenn die Base zugegeben wird. Es kann jedoch auch bevorzugt sein, dass der Katalysator erst zu einem späteren Zeitpunkt zur Mischung hinzugegeben wird, beispielsweise nach abgeschlossener Zugabe der Base.

Es soll der Deutlichkeit wegen angemerkt werden, dass unter dem Begriff "Katalysator" im Rahmen der vorliegenden Anmeldung sowohl eine tatsächlich katalytisch aktive Spezies als auch eine Katalysatorvorstufe verstanden wird, welche in der Reaktionsmischung die katalytisch aktive Spezies bildet.

Bevorzugte Katalysatoren im Rahmen der vorliegenden Anmeldung sind homogene Katalysatoren. Unter homogenen Katalysatoren werden solche Katalysatoren verstanden, welche im Reaktionsmedium gelöst vorliegen.

Der Katalysator wird bevorzugt in einer Menge von 0.001 bis 10.0 mol-% eingesetzt, besonders bevorzugt 0.01 bis 5.0 mol-%, ganz besonders bevorzugt 0.1 bis 2.0 mol-%.

Der Katalysator umfasst bevorzugt ein oder mehrere Metalle sowie einen oder mehrere Liganden. Der Katalysator kann als eine einzige Verbindung, umfassend sowohl das Metall als auch einen oder mehrere Liganden, zugegeben werden. Alternativ kann der Katalysator in situ in der Reaktionsmischung aus separat zugegebener Metallverbindung und Ligandverbindung gebildet werden.

In einer alternativen Ausführungsform wird ausschließlich eine Metallverbindung oder elementares Metall als Katalysator verwendet, ohne den Einsatz von Liganden.

Bevorzugte Verbindungen als Katalysatorbestandteile oder als eigenständige Katalysatoren ohne zusätzliche Liganden sind ausgewählt aus PdCl₂, Pd(OAc)₂, (CH₃CN)₂PdCl₂, Pd(PPh₃)₄, Bis(dibenzylidenaceton)-dipalladium (Pd₂(dba)₂), Tris(dibenzylidenaceton)dipalladium (Pd₂(dba)₃). Weiterhin können Pd/C, polymerstabilisiertes Pd sowie Raney-Nickel verwendet werden.

Bevorzugte Liganden sind ausgewählt aus monodentaten und oligodentaten Liganden. Gemäß einer bevorzugten Ausführungsform werden chelatisierende Liganden verwendet. Bevorzugte Liganden sind ausgewählt aus Phosphinen, Aminen, Aminophosphinen und N-heterocyclischen Carbenen. Bevorzugte Phosphine und Amine zur Verwendung als Liganden sind in WO 2011/008725, WO 2006/074315 und US 6307087 offenbart. Bevorzugte N-heterocyclische Carbene zur Verwendung als Liganden sind in CA 2556850 offenbart. Besonders bevorzugte N-heterocyclische Carbene sind die in der genannten Anmeldung offenbarten Carbene als Liganden der Strukturen 1a bis 1n, wie dort definiert.

Besonders bevorzugte Phosphine als Liganden sind ausgewählt aus Dicyclohexylphosphino-2',6'-dimethoxybiphenyl, Dicyclohexylphosphino-2',6'-di-isopropoxybiphenyl, Di-tert-butyl(2',4',6'-triisopropyl-3,6-dimethoxybiphenyl-2- yl)phosphin, Dicyclohexyl(2',4',6'-triisopropyl-3,6-dimethoxybiphenyl-2- yl)phosphin, Trimethylphosphin, Triethylphosphin, Tripropylphosphin, Triisopropylphosphin, Tributylphosphin, Tri-tert-butylphosphin, Tricyclohexylphosphin, Triphenylphosphin, Di-tert-butyl-chlorophosphin, Trimethylphosphit, Triethylphosphit, Tripropylphosphit, Triisopropylphosphit, Tributylphosphit, Tricyclohexylphosphit, Triphenylphosphin, Tri(o-tolyl)phosphin, Triisopropylphosphin, Tricyclohexylphosphin, 2,2'-Bis(diphenylphosphino)-1,1'-binaphthyl (BINAP), 1 ,2-Bis(dimethylphosphino)ethan, 1,2-Bis(diethylphosphino)ethan, 1,2-Bis(dipropylphosphino)-ethan, 1,2-Bis(diisopropylphosphino)ethan, 1,2-Bis(dibutyl-phosphino)ethan, 1,2-Bis(dicyclohexylphosphino)ethan, 1,3-Bis(dicyclohexyl-phosphino)propan, 1,3-Bis(diisopropylphosphino)propan, 1,4-Bis(diisopropyl-phosphino)-butan, 2,4-Bis(dicyclohexylphosphino)pentan und 1,1'-Bis(diphenylphosphino)ferrocen.

Besonders bevorzugte Amine als Liganden sind 2,2'-Bipyridin (bipy), 1,10-Phenanthrolin und Tetramethyl-ethylendiamin.

Das erfindungsgemäße Verfahren wird bevorzugt in flüssiger Phase durchgeführt. Es kann dabei ein beliebiges organisches Lösungsmittel eingesetzt werden, insbesondere solche, die durch die verwendete Base nicht oder nur in geringem Umfang deprotoniert werden. Bevorzugt sind die dem Fachmann auf dem Gebiet der organischen Synthese bekannten Lösungsmittel mit Eignung zur Verwendung in übergangsmetallkatalysierten Kupplungsreaktionen. Unter Eignung zur Verwendung in den erfindungsgemäßen Reaktionen wird insbesondere Inertizität gegenüber den Reaktionsbedingungen verstanden.

Besonders bevorzugte Lösungsmittel sind ausgewählt aus Benzol, Toluol, 1,2-Xylol, 1,3-Xylol, 1,4-Xylol, Mesitylen, Tetrahydrofuran (THF), 1,4-Dioxan, Dimethoxyethan (dme) und Bis(2-methoxyethyl)ether (diglyme). Bevorzugt werden wasserfreie Lösungsmittel eingesetzt.

Die Reaktion kann unter Normaldruck oder unter erhöhtem Druck durchgeführt werden.

Gemäß einer bevorzugten Ausführungsform wird die Reaktion unter Inertgasatmosphäre durchgeführt, beispielsweise unter Stickstoff- oder Argonatmosphäre.

Die Reaktionsdauer beträgt typischerweise zwischen wenigen Minuten und einigen Tagen, bevorzugt zwischen wenigen Minuten und 100 Stunden, ganz besonders bevorzugt zwischen 15 Minuten und 80 Stunden. Die angegebenen Zeiten beziehen sich auf die Gesamtdauer der Reaktion.

Die Reaktionstemperatur beträgt bevorzugt zwischen 0 und 300 °C, besonders bevorzugt zwischen 20 und 200 °C, ganz besonders bevorzugt zwischen 60 und 150°C und noch stärker bevorzugt zwischen 100 und 130 °C. Die angegebenen Reaktionstemperaturen beziehen sich auf die Hauptphase der Reaktion, während der alle Komponenten in der Mischung vorliegen und die Bindungsknüpfung unter Katalyse stattfindet.

Typischerweise wird zu Beginn der Reaktion die Base in einem Lösungsmittel gelöst zur Mischung hinzugegeben. Dabei wird als Lösungsmittel bevorzugt eines der für die Reaktion als bevorzugt angegebenen Lösungsmittel verwendet. Die Zugabe erfolgt bevorzugt schrittweise, besonders bevorzugt tropfenweise über einen Zeitraum von wenigstens 5 Minuten. Während dieser Phase der Reaktion ist die Reaktionstemperatur bevorzugt deutlich niedriger als während der Hauptphase der Reaktion. Besonders bevorzugt liegt sie während dieser Phase der Reaktion zwischen -70°C und 40°C, ganz besonders bevorzugt zwischen -20 °C und 30 °C, am stärksten bevorzugt zwischen 0 °C und 25 °C.

Die allgemeine Reaktionsführung und Aufarbeitung sowie die verwendeten Geräte und Reaktionsgefäße werden nicht weiter spezifiziert. Der Fachmann kann unter Beachtung der Ausführungsbeispiele und unter Hinzuziehung seines allgemeinen Fachwissens geeignete Ausführungsformen auswählen. Insbesondere geeignet sind solche Methoden, wie sie dem Fachmann allgemein für Hartwig-Buchwald-Kupplungen und ähnliche Reaktionen bekannt sind.

Gemäß einer bevorzugten Variante der Reaktionsführung werden zunächst die Aminoverbindung, die Arylverbindung sowie der Katalysator im Lösungsmittel gelöst oder suspendiert. Anschließend wird die Base zur Mischung hinzugegeben, bevorzugt schrittweise, wie oben angegeben.

Gemäß einer weiteren Variante der Reaktionsführung wird die Aminoverbindung mit einer Base versetzt, bevor die Arylverbindung in der Reaktionsmischung vorliegt. Die Arylverbindung wird in einem späteren Schritt hinzugegeben.

Diese Variante des erfindungsgemäßen Verfahrens kann wie folgt schematisch dargestellt werden: 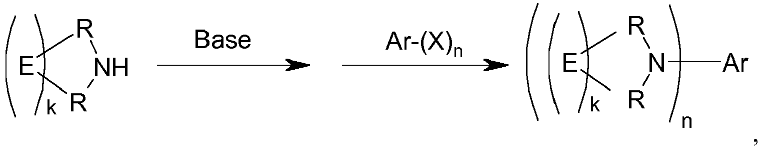 wobei die auftretenden Gruppen wie oben definiert sind. Es gelten in dieser Variante in Bezug auf die Edukte, die Base und die Katalysatorverbindung dieselben Bevorzugungen wie oben angegeben.

Gemäß dieser Variante des erfindungsgemäßen Verfahrens werden zunächst die Aminoverbindung und die Base miteinander zur Reaktion gebracht. Dabei ist die Arylverbindung noch nicht in der Mischung vorhanden. Diese Phase wird als die erste Phase der Reaktion bezeichnet. Während dieser Phase kann der Katalysator bereits in der Mischung vorhanden sein.

In einer zweiten Phase der Reaktion wird die Arylverbindung zugegeben. Bevorzugt wird auch der Katalysator erst in dieser Phase der Reaktion zugegeben, er kann jedoch auch bereits vorher der Mischung zugegeben worden sein.

Die im erfindungsgemäßen Verfahren erhaltenen Produkte werden bevorzugt als Funktionsmaterialien in elektronischen Vorrichtungen eingesetzt. Dabei sind die elektronischen Vorrichtungen bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Bevorzugt werden die erhaltenen Verfahrensprodukte als Lochtransportmaterialien und/oder als Singulett-Emittermaterialien und/oder als Elektronenblockiermaterialien und/oder als Matrixmaterialien in elektronischen Vorrichtungen verwendet. Es sind jedoch auch andere Verwendungen möglich, abhängig vom Grundkörper der Produkte und den zusätzlich zur Aminogruppe vorhandenen Substituenten.

Gegenstand der vorliegenden Erfindung ist weiterhin die Verwendung einer Base mit einem pKa-Wert von mindestens 33 bezogen auf Dimethylsulfoxid in einer übergangsmetallkatalysierten Kupplungsreaktion zwischen einer Aminoverbindung und einer Arylverbindung.

Die Aminoverbindung und die Arylverbindung sind dabei wie oben angegeben definiert.

Bevorzugt weist die Base dabei einen pKₐ-Wert bezogen auf Dimethylsulfoxid von mindestens 34, besonders bevorzugt von mindestens 36, ganz besonders bevorzugt von mindestens 38 und am stärksten bevorzugt von mindestens 40 auf.

Weiterhin ist es bevorzugt, dass die bei der Kupplungsreaktion erhaltenen Verbindungen Funktionsmaterialien für elektronische Vorrichtungen darstellen, besonders bevorzugt Funktionsmaterialien für organische Elektrolumineszenzvorrichtungen (OLEDs).

Die erfindungsgemäße Verwendung einer Base mit einem pKa-Wert bezogen auf Dimethylsulfoxid von mindestens 33 führt zu einer hervorragenden Reinheit der erhaltenen Verbindungen. Dies ist von hoher Bedeutung für den Einsatz der Verbindungen als Funktionsmaterialien in elektronischen Vorrichtungen. Weiterhin wird die Bildung von Nebenprodukten, welche sich bereits in geringer Menge abträglich auf die Leistungsdaten der elektronischen Vorrichtung auswirken, durch die Verwendung einer solchen Base zurückgedrängt.

Allgemein ist es für die Verwendung von organischen Verbindungen in elektronischen Vorrichtungen von größter Bedeutung, dass die Verbindungen in hoher Reinheit hergestellt werden können. Weiterhin ist es für eine industrielle Anwendung erforderlich, dass die Verbindungen in großem Maßstab, bevorzugt von mehr als 100 g, besonders bevorzugt von mehr als einem Kilogramm, hergestellt werden können.

Um diese Ziele zu erreichen, ist eine hohe Robustheit und Effizienz des Syntheseverfahrens unabdingbar. Insbesondere sind eine hohe Ausbeute an Produkt und eine möglichst geringer Bildung von Nebenprodukten wünschenswert.

Das erfindungsgemäße Verfahren zeichnet sich unter anderem dadurch aus, dass eine hohe Ausbeute an Produkt und eine geringe Bildung von Nebenprodukten auftritt. Insbesondere wird bei der Verwendung von sterisch anspruchsvollen Aminoverbindungen und Arylverbindungen als Edukte im erfindungsgemäßen Verfahren nur wenig Nebenprodukt in Form einer Defunktionalisierung oder einer Kupplung der verwendeten Base beobachtet. Besonders bevorzugt wird eine Ausbeute an Produkt von mehr als 80 % erzielt, und es treten weniger als 20 % Nebenprodukte, bezogen auf die Menge an eingesetzten Edukten, auf. Ganz besonders bevorzugt beträgt die Ausbeute an erhaltenem Produkt mehr als 90 %, und es treten weniger als 10% Nebenprodukte, bezogen auf die Menge an eingesetzten Edukten, auf.

Weiterhin kann das Verfahren mit einer geringen Menge an eingesetztem Katalysator und unter milden Reaktionsbedingungen durchgeführt werden.

Die folgenden Ausführungsbeispiele dienen der Verdeutlichung und detaillierten Beschreibung der Erfindung.

### Ausführungsbeispiele

### A) Vergleichs-Beispiel 1:

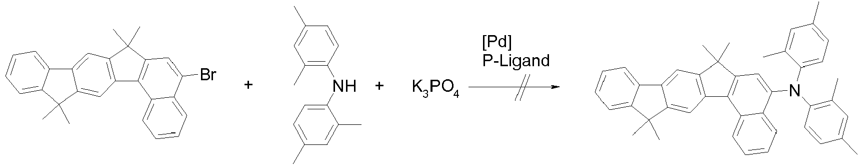

Eine Mischung aus gepulvertem Tri-Kaliumphosphat (1.2 Äq.), Bis(2,4-dimethyl-phenyl)amin (1.25 Äq), 1,2-Benzo-3-brom-6,6,12,12-tetramethyl-6,12-dihydro-indeno-[1,2-b]fluoren (1 Äq), Pd(OAc)₂ (0.02 Äq) und 2-(Dicyclohexyl-phosphino)-2',6'-dimethoxy)biphenyl (S-Phos, 0.04 Äq) in Toluol wird für 60 h unter Rückfluss erhitzt. Die DC-Kontrolle der Reaktionsmischung zeigt keine Umsetzung.

Weitere Beispiele unter Variation von Ligand und Base innerhalb der Klasse von Carbonat- und Phosphatsalzen:

| **Pd-Quelle** | **[P]-Ligand** | **Base** | **Ar** | **Ausbeute [%]** |
|---|---|---|---|---|
| Pd(OAc)₂ | S-Phos | K₃PO₄ | 2,4-dimethylphenyl | 0 |
| Pd(OAc)₂ | S-Phos | K₂CO₃ | 2,4-dimethylphenyl | 0 |
| Pd(OAc)₂ | S-Phos | Cs₂CO₃ | 2,4-dimethylphenyl | 0 |
| Pd(OAc)₂ | Ru-Phos | K₃PO₄ | 2,4-dimethylphenyl | 0 |
| Pd(OAc)₂ | Ru-Phos | K₂CO₃ | 2,4-dimethylphenyl | 0 |
| Pd(OAc)₂ | Ru-Phos | Cs₂CO₃ | 2,4-dimethylphenyl | 0 |

### B) Vergleichs-Beispiel 2:

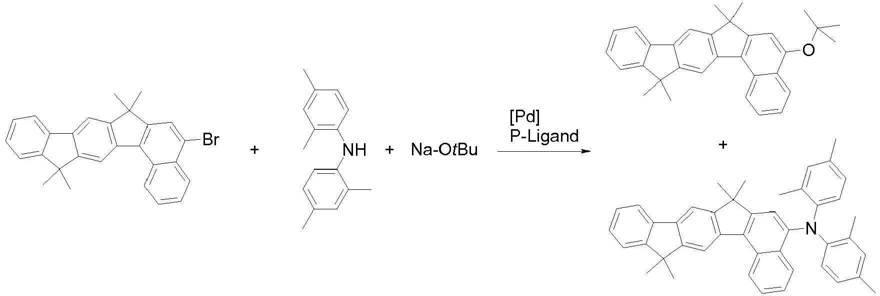

Eine Mischung aus Natrium-tert-butanolat (1.2 Äq.), Bis(2,4-dimethylphenyl)amin (1.25 Äq), 1,2-Benzo-3-brom-6,6,12,12-tetramethyl-6,12-dihydro-indeno-[1,2-b]fluoren (1 Äq), Pd(OAc)₂ (0.02 Äq) und 2-(Dicyclohexyl-phosphino)-2',6'-dimethoxy)biphenyl (S-Phos, 0.04 Äq) in Toluol wird für 2 h unter Rückfluss erhitzt. Nach dem Abkühlen auf Raumtemperatur wird mit Wasser versetzt, die organische Phase abgetrennt, mit MgSO₄ getrocknet und über Kieselgel filtriert. Das Lösemittel wird entfernt. Das 1H-NMR Spektrum des Rückstands zeigt Produkttyp A : Nebenprodukttyp NA = 17 : 80.

Weitere Beispiele unter Variation von Ligand und Edukt-Typ:

| **Edukt-typ** | **Pd-Quelle** | **[P]-Ligand** | **Base** | **Ar** | **Ausbeute [%]** | **Reinheit [%]** |
|---|---|---|---|---|---|---|
| A | Pd(OAc)₂ | S-Phos | NaO*t*Bu | 2,4-dimethylphenyl | Roh | 17 (A): 80 (NA) |
| A | Pd(OAc)₂ | Ru-Phos | NaO*t*Bu | 2,4-dimethylphenyl | Roh | 18 (A): 79 (NA) |
| A | Pd(OAc)₂ | PPh₃ | NaO*t*Bu | 2,4-dimethylphenyl | Roh | 15 (A): 40 (NA) |
| A | Pd(OAc)₂ | PCl*t*Bu₂ | NaO*t*Bu | 2,4-dimethylphenyl | 34 | 55(A): 18(NA) |
| A | Pd(OAc)₂ | P*t*Bu₃ | NaO*t*Bu | 2,4-dimethylphenyl | 66 | 96.8 (A) |
| A | Pd(OAc)₂ | P*t*Bu₃ | NaO*t*Bu | 2-methylphenyl | 64 | 96.7 (A) |
| B | Pd(OAc)₂ | P*t*Bu₃ | NaO*t*Bu | 2,4-dimethylphenyl | 27 | 97.3 (B) |
| B | Pd(OAc)₂ | P*t*Bu₃ | NaO*t*Bu | 2,3-dimethylphenyl | 20 | 98.7 (B) |
| B | Pd(OAc)₂ | PCl*t*Bu₂ | NaO*t*Bu | 2,3-dimethylphenyl | n. a. | 5 (B): 90 (NB) |

Erläuterung der Edukt-/Produktbezeichnungen:

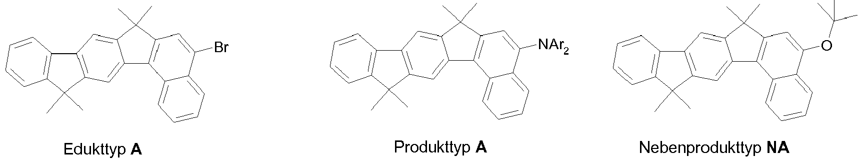

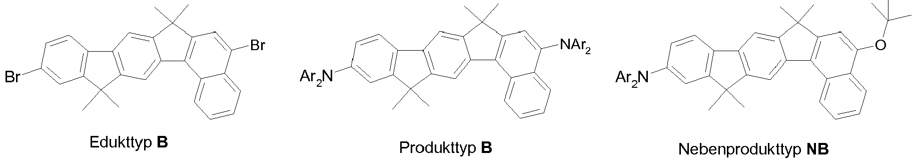

### C) Erfindungsgemäßes Beispiel:

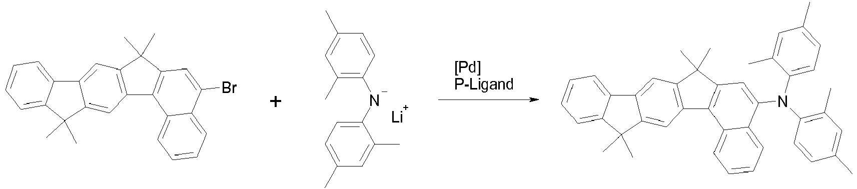

Hexyl-Lithium (2.5 M in Hexan, 1.2 Äq.) wird zu einer Lösung aus Bis(2,4-dimethyl-phenyl)amin (1.25 Äq), 1,2-Benzo-3-brom-6,6,12,12-tetramethyl-6,12-dihydro-indeno-[1,2-b]fluoren (1 Äq), Pd(OAc)₂ (0.02 Äq) und 2-(Dicyclohexyl-phosphino)-2',6'-dimethoxy)biphenyl (S-Phos, 0.04 Äq) in Toluol getropft und anschliessend für 1 h unter Rückfluss erhitzt. Nach dem Abkühlen auf Raumtemperatur wird mit Wasser versetzt, die organische Phase abgetrennt, mit MgSO₄ getrocknet und über Kieselgel filtriert. Das Lösemittel wird entfernt und der Rückstand mit Acetonitril und Isopropanol gewaschen. Ausbeute: 90%; Reinheit: 99.2% (HPLC). Die Reinheit kann durch Soxhlett-Extraktion und Sublimation auf 99.95% gesteigert werden.

Weitere Beispiele mit Hexyl-Lithium oder LDA als Base:

| **Edukt-typ** | **Pd-Quelle** | **[P]-Ligand** | **Base** | **Ar1** | **Ar2** | **Ausbeute [%]** | **Reinheit [%]** |
|---|---|---|---|---|---|---|---|
| A | Pd(OAc)₂ | S-Phos | Hex-Li | 2-methylphenyl | 2-methylphenyl | 93 | 99.3 |
| A | Pd(OAc)₂ | S-Phos | Hex-Li | 2,3-dimethylphenyl | 2,3-dimethylphenyl | 94 | 99.2 |
| A | Pd(OAc)₂ | S-Phos | Hex-Li | 2,4-dimethylphenyl | 2,4-dimethylphenyl | 90 | 99.2 |
| A | Pd(OAc)₂ | PPh₃ | Hex-Li | 2,4-dimethylphenyl | 2,4-dimethylphenyl | 79 | 98.5 |
| A | Pd(OAc)₂ | P(o-tol)₃ | Hex-Li | 2,4-dimethylphenyl | 2,4-dimethylphenyl | 88 | 98.0 |
| A | Pd(OAc)₂ | PCl*t*Bu₂ | Hex-Li | 2,4-dimethylphenyl | 2,4-dimethylphenyl | 90 | 99.0 |
| A | Pd(OAc)₂ | P*t*Bu₃ | Hex-Li | 2,4-dimethylphenyl | 2,4-dimethylphenyl | 94 | 99.5 |
| A | Pd(PPh₃)₄ | - | Hex-Li | 2,4-dimethylphenyl | 2,4-dimethylphenyl | 81 | 98.8 |
| A | Pd(OAc)₂ | S-Phos | Hex-Li | 2,4-dimethylphenyl | 2,4,6-trimethylphenyl | 88 | 99.0 |
| A | Pd(OAc)₂ | S-Phos | LDA | 2-methylphenyl | 2-methylphenyl | 92 | 99.1 |
| A | Pd(OAc)₂ | S-Phos | LDA | 2,3-dimethylphenyl | 2,3-dimethylphenyl | 90 | 99.2 |
| A | Pd(OAc)₂ | S-Phos | LDA | 2,4-dimethylphenyl | 2,4-dimethylphenyl | 87 | 99.1 |
| B | Pd(OAc)₂ | S-Phos | Hex-Li | 2,3-dimethylphenyl | 2,3-dimethylphenyl | 92 | 98.5 |
| B | Pd(OAc)₂ | S-Phos | Hex-Li | 2,4-dimethylphenyl | 2,4-dimethylphenyl | 92 | 98.9 |

Erläuterung der Bezeichnungen der Edukttypen: s. o.

### D) pK_{a'}-Werte der verwendeten Basen (K₃PO₄, K₂CO₃ und Cs₂CO₃ sind sehr schwache Basen):

| | pKₐ (DMSO) |
|---|---|
| NaOtBu (Vergleichsbeispiel) | 29 |
| LDA (erfindungsgemäßes Beispiel) | 44 |
| Hexyl-Lithium (erfindungsgemäßes Beispiel) | ca. 50 |

## Patentansprüche

1. Verfahren zur Herstellung einer Verbindung enthaltend mindestens eine Arylaminogruppe, **dadurch gekennzeichnet, dass** das Verfahren mindestens eine übergangsmetallkatalysierte Kupplungsreaktion zwischen einer Aminoverbindung und einer Arylverbindung umfasst, bei der eine Base mit einem pKₐ-Wert bezogen auf Dimethylsulfoxid von mindestens 33 eingesetzt wird, wobei die Base aus Amiden mit Alkali- oder Erdalkalimetallgegenion und Organometallverbindungen mit formal negativ geladenem Kohlenstoffatom mit Alkalimetall- oder Erdalkalimetall-Gegenion gewählt ist, und wobei das Verfahren folgendem Schema entspricht: 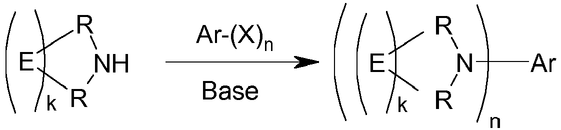 wobei für die auftretenden Symbole und Indices gilt:
Ar ist ein aromatisches Ringsystem mit 10 bis 30 aromatischen Ringatomen, das mit einem oder mehreren Resten R¹ substituiert sein kann, und welches mindestens zwei miteinander kondensierte aromatische Ringe aufweist, wobei:
- in der Arylaminogruppe, der eine aromatische Ring in einer ortho-Position zur Bindung an das Stickstoffatom an den anderen aromatischen Ring ankondensiert ist; und
- in der Verbindung Ar-(X)ₙ, der eine aromatische Ring in einer ortho-Position zur Bindung an die X Gruppe an den anderen aromatischen Ring ankondensiert ist;
E ist bei jedem Auftreten gleich oder verschieden gewählt aus einer Einfachbindung, C(R¹)₂, C=O, Si(R¹)₂, NR¹, O und S;
R ist gleich Phenyl, das durch einen oder mehrere Reste R¹ substituiert sein kann;
R¹ ist, gleich oder verschieden, bei jedem Auftreten ausgewählt aus H, D, F, Cl, Br, I, B(OR²)₂, CHO, C(=O)R², CR²=C(R²)₂, CN, C(=O)OR², C(=O)N(R²)₂, Si(R²)₃, N(R²)₂, NO₂, P(=O)(R²)₂, OSO₂R², OR², S(=O)R², S(=O)₂R², einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenyl- oder Alkinylgruppe mit 2 bis 20 C-Atomen, wobei die oben genannten Gruppen jeweils mit einem oder mehreren Resten R² substituiert sein können und wobei eine oder mehrere CH₂-Gruppen in den oben genannten Gruppen durch -R²C=CR²⁻, -C≡C-, Si(R²)₂, C=O , C=S, C=NR², -C(=O)O-, -C(=O)NR²-, NR², P(=O)(R²), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches Ringsystem mit 5 bis 30 aromatischen Ringatomen, das jeweils durch einen oder mehrere Reste R² substituiert sein kann, oder eine Aryloxygruppe mit 5 bis 30 aromatischen Ringatomen, die durch einen oder mehrere Reste R² substituiert sein kann, wobei zwei oder mehr Reste R¹ miteinander verknüpft sein können und einen Ring bilden können;
R² ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R² miteinander verknüpft sein und einen Ring bilden;
X ist bei jedem Auftreten gleich oder verschieden eine beliebige Abgangsgruppe;
k ist bei jedem Auftreten gleich oder verschieden 0 oder 1, wobei, wenn k = Null ist, die Gruppe E nicht vorhanden ist;
i ist bei jedem Auftreten gleich oder verschieden einen Wert von 0 bis 5, wenn k = Null ist, oder einen Wert von 0 bis 4, wenn k = 1; und
n ist 1;
wobei die Verbindung 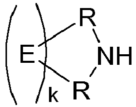 die Aminoverbindung darstellt, die Verbindung der Formel
Ar-(X)ₙ
die Arylverbindung darstellt, und die Verbindung 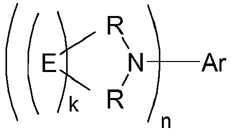 die Verbindung, welche mindestens eine Arylaminogruppe enthält, darstellt,
und wobei ein Katalysator vorhanden ist, wobei der Katalysator eine Verbindung darstellt, welche mindestens ein Metall aus Palladium enthält.

2. Verfahren nach Anspruch 1, **dadurch gekennzeichnet, dass** die Base einen pKₐ-Wert aufweist, welcher mindestens um den Betrag von 6 größer ist als der pKₐ-Wert der Aminoverbindung.

3. Verfahren nach Anspruch 1 oder 2, **dadurch gekennzeichnet, dass** die Base einen pKₐ-Wert bezogen auf Dimethylsulfoxid von mindestens 34, besonders bevorzugt von mindestens 36 aufweist.

4. Verfahren nach einem oder mehreren der Ansprüche 1 bis 3, **dadurch gekennzeichnet, dass** die Gruppe Ar eine kondensierte Arylgruppe ausgewählt aus Naphthalin, Anthracen, Phenanthren, Pyren, Chrysen, Perylen, Fluoranthen, Benzanthracen, Benzphenanthren, Tetracen, Pentacen, Benzpyren, Acridin und Phenanthridin umfasst oder dass die Gruppe Ar ein aromatisches Ringsystem ausgewählt aus Fluoren, Spirobifluoren, cis- oder trans-Indenofluoren, cis- oder trans-Indolocarbazol, cis- oder trans-Indenocarbazol, cis- oder trans-Monobenzindenofluoren und cis- oder trans-Dibenzindenofluoren umfasst.

## Claims

1. Process for the preparation of a compound containing at least one arylamino group, **characterised in that** the process comprises at least one transition-metal-catalysed coupling reaction between an amino compound and an aryl compound, in which a base having a pKa value, based on dimethyl sulfoxide, of at least 33 is employed, where the base is selected from amides with alkali-metal or alkaline-earth metal counterion and organometallic compounds containing a formally negatively charged carbon atom with alkali-metal or alkaline-earth metal counterion, and where the process corresponds to the following scheme: 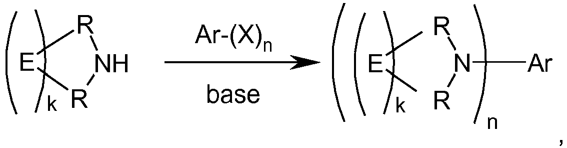 where the following applies to the symbols and indices occurring:
Ar is an aromatic ring system having 10 to 30 aromatic ring atoms, which may be substituted by one or more radicals R¹, and which contains at least two aromatic rings condensed with one another, where:
- in the arylamino group, the one aromatic ring is condensed onto the other aromatic ring in an ortho position to the bond to the nitrogen atom; and
- in the compound Ar-(X)ₙ, the one aromatic is condensed onto the other aromatic ring in an ortho position to the bond to the X group;
E is selected on each occurrence, identically or differently, from a single bond, C(R¹)₂, C=O, Si(R¹)₂, NR¹, O and S;
R is equal to phenyl, which may be substituted by one or more radicals R¹_{;}
R¹ is selected on each occurrence, identically or differently, from H, D, F, Cl, Br, I, B(OR²)₂, CHO, C(=O)R², CR²=C(R²)₂, CN, C(=O)OR², C(=O)N(R²)₂, Si(R²)₃, N(R²)₂, NO₂, P(=O)(R²)₂, OSO₂R², OR², S(=O)R², S(=O)₂R², a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may in each case be substituted by one or more radicals R² and where one or more CH₂ groups in the above-mentioned groups may be replaced by -R²C=CR²⁻, -C≡C-, Si(R²)₂, C=O, C=S, C=NR², -C(=O)O-, -C(=O)NR²-, NR², P(=O)(R²), -O-, -S-, SO or SO₂ and where one or more H atoms in the above-mentioned groups may be replaced by D, F, Cl, Br, I, CN or NO₂, or an aromatic ring system having 5 to 30 aromatic ring atoms, which may in each case be substituted by one or more radicals R², or an aryloxy group having 5 to 30 aromatic ring atoms, which may be substituted by one or more radicals R², where two or more radicals R¹ may be linked to one another and may form a ring;
R² is on each occurrence, identically or differently, H, D, F or an aliphatic, aromatic or heteroaromatic organic radical having 1 to 20 C atoms, in which, in addition, one or more H atoms may be replaced by D or F; two or more substituents R² may be linked to one another and may form a ring;
X is on each occurrence, identically or differently, any desired leaving group;
k is on each occurrence, identically or differently, 0 or 1, where the group E is not present if k = zero;
i is on each occurrence, identically or differently, a value from 0 to 5 if k = zero, or a value from 0 to 4 if k = 1; and
n is 1;
where the compound 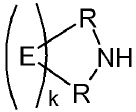 represents the amino compound, the compound of the formula
Ar-(X)ₙ
represents the aryl compound, and the compound 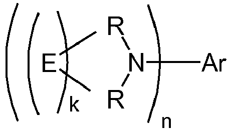 represents the compound which contains at least one arylamino group,
and where a catalyst is present, where the catalyst is a compound which contains at least one metal from palladium.

2. Process according to Claim 1, **characterised in that** the base has a pKₐ value which is greater than the pKₐ value of the amino compound by an amount of at least 6.

3. Process according to Claim 1 or 2, **characterised in that** the base has a pKₐ value, based on dimethyl sulfoxide, of at least 34, particularly preferably of at least 36.

4. Process according to one or more of Claims 1 to 3, **characterised in that** the group Ar comprises a condensed aryl group selected from naphthalene, anthracene, phenanthrene, pyrene, chrysene, perylene, fluoranthene, benzanthracene, benzophenanthrene, tetracene, pentacene, benzopyrene, acridine and phenanthridine or **in that** the group Ar comprises an aromatic ring system selected from fluorene, spirobifluorene, cis- or trans-indenofluorene, cis- or trans-indolocarbazole, cis- or trans-indenocarbazole, cis- or trans-monobenzindenofluorene and cis- or trans-dibenzindenofluorene.

## Revendications

1. Procédé de préparation d'un composé contenant au moins un groupement arylamino, **caractérisé en ce que** le procédé comprend au moins une réaction de couplage catalysée par un métal de transition entre un composé amino et un composé aryle, où on emploie une base ayant une valeur de pKₐ, sur la base du diméthylsulfoxyde, d'au moins 33, où la base est choisie parmi les amides ayant un contre-ion de métal alcalin ou de métal alcalino-terreux et les composés organométalliques contenant un atome de carbone négativement chargé de manière formelle ayant un contre-ion de métal alcalin ou alcalino-terreux, et où le procédé correspond au schéma suivant : 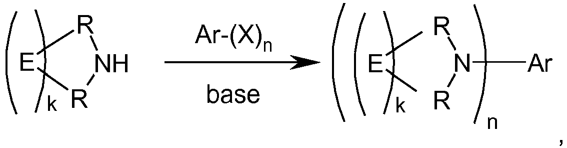 où ce qui suit s'applique aux symboles et aux indices étant rencontrés :
Ar est un noyau aromatique ayant de 10 à 30 atomes de cycle aromatique, qui peut être substitué par un ou plusieurs radicaux R¹, et qui contient au moins deux cycles aromatiques condensés l'un avec l'autre, où :
- dans le groupement arylamino, l'un des cycles aromatiques est condensé avec l'autre cycle aromatique en position ortho par rapport à la liaison à l'atome d'azote ; et
- dans le composé Ar-(X)ₙ, l'un des cycles aromatiques est condensé avec l'autre cycle aromatique en position ortho par rapport à la liaison au groupement X ;
E est choisi à chaque occurrence, de manière identique ou différente, parmi une liaison simple, C(R¹)₂, C=O, Si(R¹)₂, NR¹, O et S ;
R est égal à phényle, qui peut être substitué par un ou plusieurs radicaux R¹ ;
R¹ est choisi à chaque occurrence, de manière identique ou différente, parmi H, D, F, Cl, Br, I, B(OR²)₂, CHO, C(=O)R², CR²=C(R²)₂, CN, C(=O)OR², C(=O)N(R²)₂, Si(R²)₃, N(R²)₂, NO₂, P(=O)(R²)₂, OSO₂R², OR², S(=O)R², S(=O)₂R², un groupement alkyle, alcoxy ou thioalkyle à chaîne linéaire ayant de 1 à 20 atomes de C ou un groupement alkyle, alcoxy ou thioalkyle ramifié ou cyclique ayant de 3 à 20 atomes de C ou un groupement alcényle ou alcynyle ayant de 2 à 20 atomes de C, où les groupements susmentionnés peuvent dans chaque cas être substitués par un ou plusieurs radicaux R² et où un ou plusieurs groupements CH₂ dans les groupements susmentionnés peuvent être remplacés par -R²C=CR²⁻, -C≡C-, Si(R²)₂, C=O, C=S, C=NR², -C(=O)O-, -C(=O)NR²-, NR², P(=O)(R²), -O-, -S-, SO ou SO₂ et où un ou plusieurs atomes de H dans les groupements susmentionnés peuvent être remplacés par D, F, Cl, Br, I, CN ou NO₂, ou un noyau aromatique ayant de 5 à 30 atomes de cycle aromatique, qui peut dans chaque cas être substitué par un ou plusieurs radicaux R², ou un groupement aryloxy ayant de 5 à 30 atomes de cycle aromatique, qui peut être substitué par un ou plusieurs radicaux R², où deux, ou plus, radicaux R¹ peuvent être liés l'un avec l'autre et peuvent former un cycle ;
R² est à chaque occurrence, de manière identique ou différente, H, D, F ou un radical organique aliphatique, aromatique ou hétéroaromatique ayant de 1 à 20 atomes de C, où, de plus, un ou plusieurs atomes de H peuvent être remplacés par D ou F ; deux, ou plus, substituants R² peuvent être liés l'un avec l'autre et peuvent former un cycle ;
X est à chaque occurrence, de manière identique ou différente, un groupement partant désiré quelconque ;
k vaut à chaque occurrence, de manière identique ou différente, 0 ou 1, où le groupement E n'est pas présent si k = zéro ;
i est à chaque occurrence, de manière identique ou différente, une valeur allant de 0 à 5 si k = zéro, ou une valeur allant de 0 à 4 si k = 1 ; et
n vaut 1 ;
où le composé 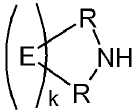 représente le composé amino, le composé de formule
Ar-(X)n
représente le composé aryle, et le composé 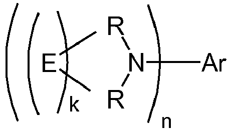 représente le composé qui contient au moins un groupement arylamino,
et où un catalyseur est présent, où le catalyseur est un composé qui contient au moins un métal à base de palladium.

2. Procédé selon la revendication 1, **caractérisé en ce que** la base possède une valeur de pKₐ qui est supérieure d'au moins 6 points par rapport à la valeur de pKₐ du composé amino.

3. Procédé selon la revendication 1 ou 2, **caractérisé en ce que** la base possède une valeur de pKₐ, sur la base du diméthylsulfoxyde, d'au moins 34, particulièrement préférablement d'au moins 36.

4. Procédé selon l'une ou plusieurs parmi les revendications 1 à 3, **caractérisé en ce que** le groupement Ar comprend un groupement aryle condensé choisi parmi le naphtalène, l'anthracène, le phénanthrène, le pyrène, le chrysène, le pérylène, le fluoranthène, le benzanthracène, le benzophénanthrène, le tétracène, le pentacène, le benzopyrène, l'acridine et la phénanthridine ou **en ce que** le groupement Ar comprend un noyau aromatique choisi parmi le fluorène, le spirobifluorène, le cis- ou trans-indénofluorène, le cis- ou trans-indolocarbazole, le cis- ou trans-indénocarbazole, le cis- ou trans-monobenzindénofluorène et le cis- ou trans-dibenzindénofluorène.
